# EUROPEAN PATENT APPLICATION

(11) **EP 4 604 131 A1**
(43) Date of publication of application: **20.08.2025**
(21) Application number: 25153459.0
(22) Date of filing: 23.01.2025
(51) Int. Cl.: G16C 60/00, G16C 20/70, G16C 20/30

(54) **METHOD FOR GENERATING RECIPES OF POLYMER COMPOSITE MATERIAL AND DEVICE THEREOF**

(30) Priority: 19.02.2024 KR 20240023727
(71) Applicant: SK Geo Centric Co., Ltd., Seoul 03188 (KR); SK innovation Co., Ltd., Seoul 03188 (KR)
(72) Inventor: JEON, Chan Woong, 03161 Seoul (KR); KWON, June Haan, 03161 Seoul (KR); KIM, Duk Hee, 03161 Seoul (KR); KIM, Seok Jun, 03161 Seoul (KR); KIM, Sun Ryong, 03161 Seoul (KR); KIM, Jeong Hwan, 34124 Daejeon (KR); KIM, Chan Woo, 03161 Seoul (KR); LEE, Joo Pyung, 03161 Seoul (KR); LEE, Hee Eun, 34124 Daejeon (KR); JUNG, Hyun Jung, 34124 Daejeon (KR)
(74) Representative: Thoma, Michael

(57) **Abstract**

According to various embodiments of the present disclosure, a method for predicting recipes of a polymer composite material and a device thereof may be provided, wherein the method includes: obtaining at least one property for a target polymer composite material; predicting a recipe including at least two materials containing at least one polymer for synthesizing the target polymer composite material and a mixing ratio for each of the two or more materials based on at least one property and recipe prediction model; and outputting the recipe.

## Description

### [BACKGROUND OF THE INVENTION]

### 1. Field of the Invention

The present disclosure relates to a method for generating recipes of a polymer composite material and a device thereof

### 2. Description of the Related Art

A polymer composite material is used in various industrial fields, and in order to predict properties according to recipes, or predict materials of a polymer composite material having target properties during the development and synthesis thereof experiments, simulations, and data-based approaches are comprehensively utilized.

The current techniques use methods that still require repeated trial and error methods in order to predict materials for the polymer composite material with target properties, for example, a method of collecting property data by trying the prediction while changing various composition ratios and process conditions in a laboratory, inferring the properties of the material based on the collected property data, and improving the mixing of materials to secure a composition of the materials is used.

Thereby, attempts are being made to predict materials for the polymer composite material with target properties using a model trained based on the artificial intelligence currently developed, but it takes a lot of costs and time to perform training of the commercially available artificial intelligence model, and there are limitations due to a reason that it is difficult to consider numerous variables and interactions between recipes and properties of the material synthesized based on the recipes.

### [SUMMARY OF THE INVENTION]

It is an object of the present disclosure to provide a method for generating recipes of a polymer composite material and a device thereof

Problems to be solved through various embodiments are not limited to the above-described problem, and other problems not described above will be clearly understood by those skilled in the art from the following description.

To achieve the above object, according to an aspect of the present disclosure, there is provided a method for predicting recipes of a polymer composite material, which includes: obtaining at least one property for a target polymer composite material; predicting a recipe including at least two materials containing at least one polymer for synthesizing the target polymer composite material and a mixing ratio for each ofthe two or more materials based on at least one property and recipe prediction model; and outputting the recipe.

Here, the recipe prediction model may be a function configured to set properties ofthe polymer composite material, which is an output of the property prediction model, as an output variable to be maximized or minimized based on a machine learning algorithm, and predict a recipe, which is an input of the property prediction model, as an input variable.

Here, the property prediction model may be an artificial intelligence model trained to input an input recipe including two or more materials containing at least one polymer and a mixing ratio for each of the two or more materials, and predict output properties of the polymer composite material according to an input of the input recipe and output them.

Here, the step ofpredicting a recipe may include: inputting the at least one property into an output variable of the recipe prediction model; calculating the two or more materials containing at least one polymer and a mixing ratio for each of the two or more materials as input variables of the recipe prediction model so that the output variable is maximized or minimized; and determining the calculated input variables as the recipe.

Here, the step of predicting may generate the recipe by including at least a portion of a first recipe including a pure polymer and a second recipe including at least one recycled polymer.

Here, when generating the second recipe, the step ofpredicting a recipe may reconstruct a mixing ratio for each of two or more materials including the recycled polymer based on a relational expression between a difference in properties of the pure polymer and the recycled polymer.

Here, the relational expression for a difference in properties may be generated in a learning process of the property prediction model which is the basis of the recipe prediction model, and the property prediction model may be an artificial intelligence model trained to input an input recipe including two or more materials containing at least one polymer and a mixing ratio for each of the two or more materials, and predict output properties of the polymer composite material according to an input of the input recipe and output them.

Here, the method may further include, verifying the recipe predicted from the recipe prediction model based on the property prediction model, which is performed between the predicting step and the outputting step.

Here, the step of verifying the recipe may include: processing the recipe as an input recipe to the property prediction model, and then comparing the output properties acquired from the property prediction model with at least one property; and determining to output the recipe for the at least one property according to the comparison result.

Here, the step of determining to output the recipe may include: determining the recipe as a recipe for the at least one property if the comparison result is within a preset error range; and performing the prediction step again if the comparison result deviates from the preset error range.

Here, the at least one property may include a value for at least one item of melt index, tensile strength, tensile failure, tearing strength, yield strength, flexural modulus, impact strength, elongation at break, heat distortion temperature, air permeability and shrinkage.

Here, the at least one polymer may include polypropylene (PP), polyethylene (PE), or polyethylene terephthalate (PET).

According to another aspect of the present disclosure, there is provided a device for predicting recipes of a polymer composite material, which includes: a property information acquisition unit configured to acquire at least one property of a target polymer composite material; a recipe prediction unit configured to predict a recipe including two or more materials containing at least one polymer for synthesizing the target polymer composite material and a mixing ratio for each of the two or more materials based on at least one property and recipe prediction model; and a result output unit configured to output the recipe.

Here, the recipe prediction model may be a function configured to set properties of the polymer composite material, which is an output of the property prediction model, as an output variable to be maximized or minimized based on a machine learning algorithm, and predict a recipe, which is an input of the property prediction model, as an input variable.

Here, the property prediction model may be an artificial intelligence model trained to input an input recipe including two or more materials containing at least one polymer and a mixing ratio for each of the two or more materials, and predict output properties of the polymer composite material according to an input of the input recipe and output them.

Here, the recipe prediction unit may input the at least one property into an output variable of the recipe prediction model, calculate two or more materials containing at least one polymer and a mixing ratio for each of the two or more materials as input variables of the recipe prediction model so that the output variable is maximized or minimized, and determine the calculated input variable as the recipe.

Here, the recipe prediction unit may generate the recipe by including at least a portion of a first recipe including a pure polymer and a second recipe including at least one recycled polymer.

Here, when generating the second recipe, the recipe prediction unit may reconstruct the mixing ratio for each of two or more materials including the recycled polymer based on a relational expression between a difference in properties of the pure polymer and the recycled polymer.

Here, the relational expression for a difference in properties may be generated in a learning process of the property prediction model which is the basis of the recipe prediction model, and the property prediction model may be an artificial intelligence model trained to input an input recipe including two or more materials containing at least one polymer and a mixing ratio for each of the two or more materials, and predict output properties of the polymer composite material according to an input of the input recipe and output them.

Here, the recipe prediction unit may verify the recipe predicted from the recipe prediction model based on the property prediction model.

Here, the recipe prediction unit may process the recipe as an input recipe to the property prediction model, and then compare the output properties acquired from the property prediction model with at least one property; and verify the recipe by determining to output it for the at least one property according to the comparison result.

Here, the recipe prediction unit may determine the recipe as a recipe for the at least one property if the comparison result is within a preset error range, and perform the prediction step again if the comparison result deviates from the preset error range.

Here, the at least one property may include a value for at least one item of melt index, tensile strength, tensile failure, tearing strength, yield strength, flexural modulus, impact strength, elongation at break, heat distortion temperature, air permeability and shrinkage, and the at least one polymer includes polypropylene (PP), polyethylene (PE), or polyethylene terephthalate (PET).

Here, the at least one polymer may include polypropylene (PP), polyethylene (PE), or polyethylene terephthalate (PET).

As described above, the method for generating recipes of a polymer composite material and the device thereof may provide an environment capable of quickly checking materials and a mixing ratio thereof for a polymer composite material only by inputting at least some properties of the desired polymer composite material.

According to various embodiments, the method for generating recipes of a polymer composite material and the device thereof allow users to easily convert and check a material mixing ratio when using a pure polymer or a material mixing ratio when including a recycled polymer by inputting the properties of the target polymer composite material, thereby significantly reducing the time and costs required for development of materials.

### [BRIEF DESCRIPTION OF THE DRAWINGS]

The above and other objects, features and other advantages of the present disclosure will be more clearly understood from the following detailed description taken in conjunction with the accompanying drawings, in which:
FIG. 1 is a block diagram schematically illustrating the configuration of a device according to an embodiment of the present disclosure;
FIG. 2 is a flowchart illustrating the flow of an operation of predicting recipes for a polymer composite material with specific properties in the device according to an embodiment;
FIG. 3 is a flowchart illustrating the detailed flow of the operation of predicting recipes for a polymer composite material with specific properties in the device according to an embodiment;
FIG. 4 is a flowchart illustrating an operation of verifying the recipe predicted in the operation of predicting recipes for a polymer composite material with specific properties in the device according to an embodiment;
FIG. 5 is a flowchart illustrating the detailed flow of the operation of verifying the predicted recipe in the device according to an embodiment;
FIG. 6 is a flowchart illustrating the detailed flow of the operation of verifying the predicted recipe in the device according to an embodiment;
FIG. 7 is a view schematically illustrating an operation of a property prediction model to output properties of the polymer composite material using the properties of the materials included in the recipe as an input in the device according to an embodiment;
FIG. 8 is a flowchart illustrating the flow of an operation of training the property prediction model based on a deep learning algorithm in the device according to an embodiment;
FIG. 9 is a view schematically illustrating an operation of the property prediction model to predict attributes of the materials using the properties of the materials included in the recipe as an input, and output properties of the polymer composite material using the attributes of the materials as an input in the device according to an embodiment;
FIG. 10 is a flowchart illustrating the flow of an operation of training the property prediction model based on the deep learning algorithm in the device according to an embodiment of the present disclosure; and
FIG. 11 is a flowchart illustrating the flow of the detailed operation of training the property prediction model based on the deep learning algorithm in the device according to an embodiment of the present disclosure.

### [DETAILED DESCRIPTION OF THE INVENTION]

Hereinafter, embodiments will be described in detail with reference to the accompanying drawings. However, since various changes may be made in the embodiments, the scope of the patent disclosure is not limited or restricted by these embodiments. It should be understood that all modifications, equivalents, and alternatives for the embodiments are included in the scope of the present disclosure.

It will be understood that when a component is described to as being "connected," "combined" or "coupled" to another component, the component may be directly connected or coupled the another component, but it may be "connected," "combined" or "coupled" to the another component intervening another component may be present.

Further, in describing the components of the embodiment, the meaning of "or" may mean each of the components, may mean two or more of the components, or may mean all of the components. For example, it should be understood that the expressions "a, b or c" represent any one of "a," "b," "c," "a and b," "a and c," "b and c," and "a, b and c."

Components included in one embodiment and components including common functions will be described using the same names in other embodiments. The description given in one embodiment may be applied to other embodiments, and therefore will not be described in detail within the overlapping range, unless there is a description opposite thereto.

The device and/or 'data' processed by the device may be expressed in terms of 'information'. Here, the information may be used as a concept including the data.

The present disclosure describes a method for generating recipes of a polymer composite material and a device thereof To describe in more detail, a method for generating recipes of a polymer composite material, which satisfy target property information input from a user as a minimum value or maximum value and a device thereof will be described.

According to various embodiments, the operation of generating recipes of a polymer composite material may be performed based on at least one deep learning algorithm. To describe in more detail, the operation of generating recipes of a polymer composite material may be performed based on at least one deep learning model and at least one optimization model.

According to an embodiment, the deep learning model may include a property prediction model trained to predict properties of a polymer composite material prepared for various properties of materials containing at least one polymer and a mixing ratio of the materials.

In addition, the optimization model may include a recipe prediction model configured to predict materials containing at least one polymer and a mixing ratio of the materials so as to satisfy the input target properties.

Here, the recipe prediction model may include at least one obj ective function generated based on at least one machine learning algorithm (or machine learning optimization algorithm). In this case, the machine learning algorithm may include at least some of optimization algorithms based on machine learning such as a Bayesian optimization algorithm, a grid search algorithm, and a gradient descent algorithm, etc.

Here, the polymer composite material may be a material prepared using at least one polymer, as well as polymer blends, polymer copolymer, polymer nanocomposites, polymer interpenetrating network (IPN), or polymer metal composites.

In addition, the polymer which is the material of the polymer composite material may include at least one polymer among polypropylene (PP), polyethylene (PE), and polyethylene terephthalate (PET).

In this case, the polymer may include polypropylene, polyethylene, and polyethylene terephthalate as polymer categories, and may include at least one polymer material for each of these categories.

Hereinafter, various embodiments of the present disclosure will be described with reference to the accompanying drawings. However, the drawings attached to the present specification serve to further understand the technical idea together with the detailed description, such that the present disclosure should not be construed as being limited only to the illustrations of the drawings.

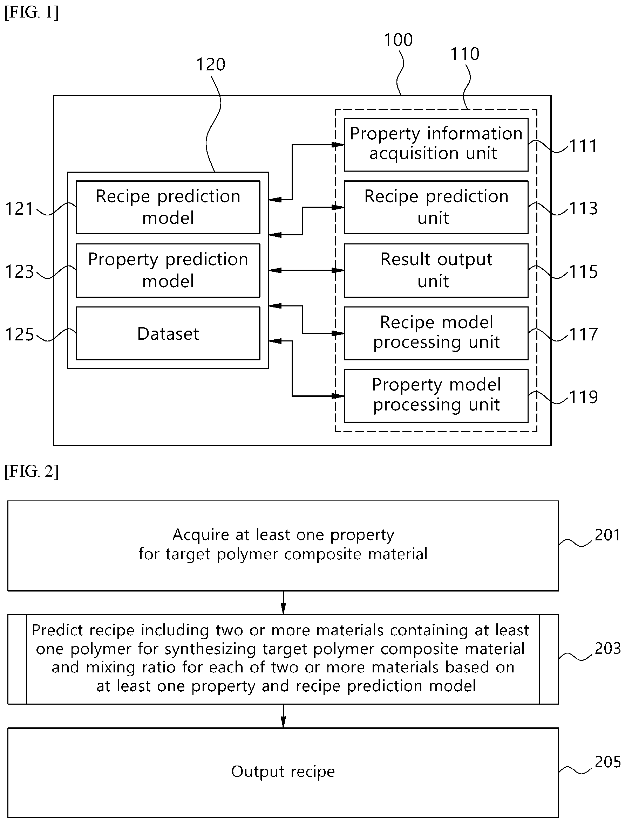

FIG. 1 is a block diagram illustrating the configuration of a device according to an embodiment. To describe in more detail, the device may be illustrated as a block diagram by dividing the detailed configuration according to functions thereof as shown in FIG. 1.

First, referring to FIG. 1, a device 100 may include: a property information acquisition unit 111 configured to acquire at least one property for a specific polymer composite material in order to predict a recipe capable of synthesizing a polymer composite material with properties using the input properties; a recipe prediction unit 113 configured to predict a recipe capable of synthesizing the polymer composite material with the properties acquired based on the acquired properties; a result output unit 115 configured to output the predicted recipe; and a recipe model processingunit 117 configured to generate and perform optimization of a recipe prediction model 121 for predicting recipes capable of synthesizing the polymer composite material with the properties using the input properties.

Here, the device 100 may further include a property model processing unit 119 configured to process a property prediction model 123 which is the basis of the recipe prediction model 121.

In this case, the property model processing unit 119 may include at least some of a recipe acquisition unit configured to acquire recipes for synthesizing the polymer composite material and acquire recipes including a plurality of materials and properties for each of the materials and a mixing ratio of the materials from the acquired recipe; a property prediction unit configured to predict properties of the polymer composite material based on the properties and mixing ratio of the acquired materials; a result output unit configured to output the predicted properties of the polymer composite material; and a model learning unit configured to perform training of the property prediction model 123.

Here, the result output unit forming the property model processing unit 119 may include a result output unit 115 which outputs the predicted recipe, or may be configured to output the properties which are predicted through an operation identical or similar to that of the result output unit 115.

According to various embodiments, the device 100 may include a storage unit 120 configured to store the recipe prediction model 121. The property prediction model 123 which is the basis ofthe recipe prediction model 121 may be stored in the storage unit 120. In addition, at least one dataset 125 used to perform training of the property prediction model 123 may be stored in the storage unit 120.

Hereinafter, as an operation of the device 100 based on FIG. 1, operations of predicting recipes for synthesizing a polymer composite material satisfying specific properties by the device 100 will be described with reference to FIGS. 2 to 13.

First, the operation of predicting recipes of a polymer composite material satisfying specific properties using the recipe prediction model will be described with reference to FIGS. 2 to 6.

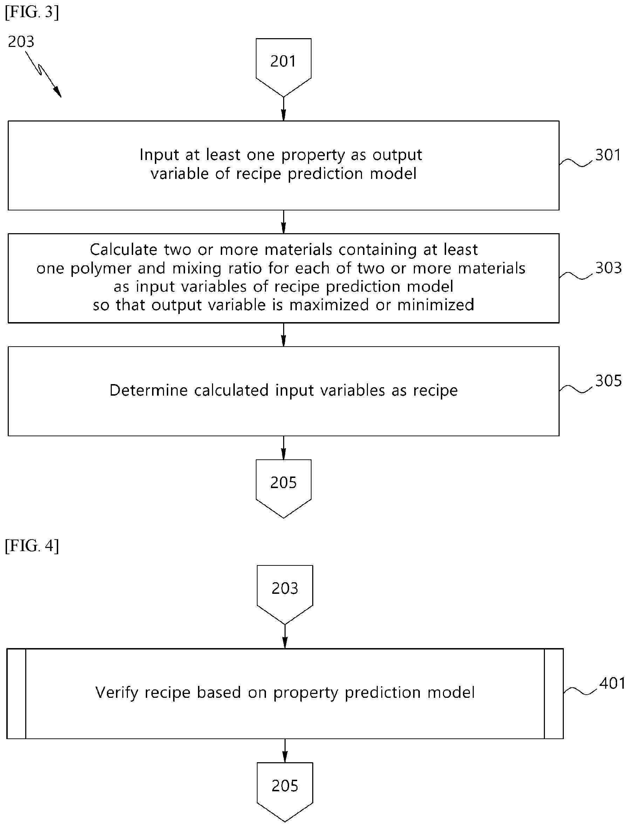

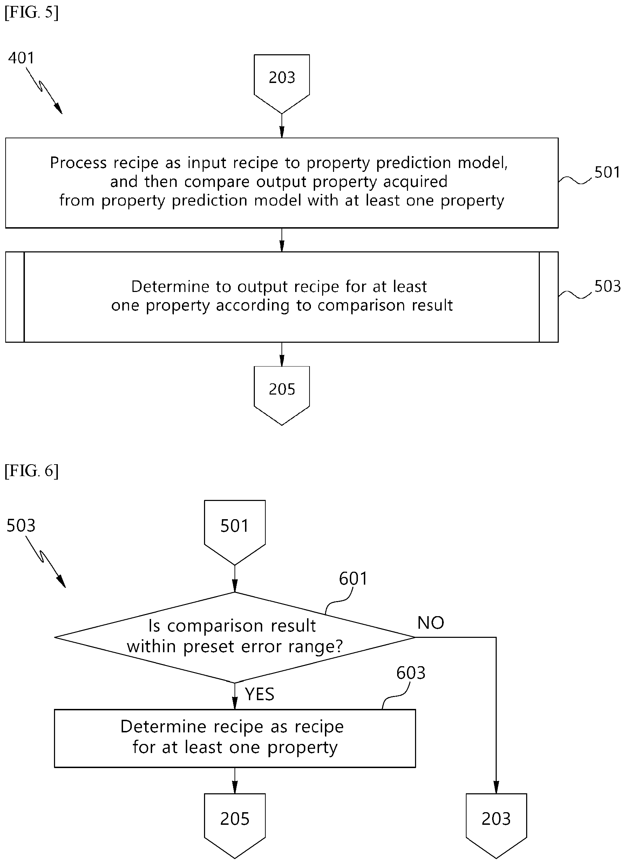

To this end, FIG. 2 is a flowchart illustrating the flow of an operation of predicting recipes for a polymer composite material with specific properties in the device according to an embodiment. FIG. 3 is a flowchart illustrating the detailed flow of the operation of predicting recipes for a polymer composite material with specific properties in the device according to an embodiment. FIG. 4 is a flowchart illustrating an operation ofverifying the recipe predicted in the operation of predicting recipes for a polymer composite material with specific properties in the device according to an embodiment. FIG. 5 is a flowchart illustrating the detailed flow of the operation of verifying the predicted recipe in the device according to an embodiment. In addition, FIG. 6 is a flowchart illustrating the detailed flow of the operation of verifying the predicted recipe in the device according to an embodiment.

Referring to FIG. 2, in step 201, the property information acquisition unit 111 may acquire at least one property for a target polymer composite material. To describe in more detail, the property information acquisition unit 111 may acquire target property values for at least one property item for the polymer composite material to be synthesized.

For example, at least one property may include at least one item of melt index, tensile strength, tensile failure, tearing strength, yield strength, flexural modulus, impact strength, elongation at break, heat distortion temperature, air permeability and shrinkage, and values for each of the items.

To describe in more detail, the impact strength may include properties of dart impact strength and/or Izod impact strength. In addition, at least some of the tearing strength, yield strength, tensile failure, and elongation at break may include properties in a machine direction and/or properties in a transverse direction.

In describing embodiments of the present disclosure, when expressing the 'properties (or at least one property) of a specific material', it may be expressed including properties (or property items) and property values of the specific material such as property items (e.g., tensile strength) and property values (tensile strength value possessed by a specific polypropylene) of the specific material (e.g., specific polypropylene).

According to an embodiment, the values for each of the acquired at least one property item may be acquired as a maximum value or a minimum value. For example, the property information acquisition unit 111 may acquire at least one property including a minimum value of 25 MPa in the tensile strength item, a minimum value of 0.2 g/10 min in the melt index item, a minimum value of 80 degrees Celsius (°C) in the heat distortion temperature item, and a minimum value of 1.5% in the shrinkage item.

According to the description, it is described that the property information acquisition unit 111 acquires at least one property including minimum values for tensile strength, melt index, heat distortion temperature and shrinkage. However, it is not limited thereto, and the property information acquisition unit 111 may acquire at least one property by including a maximum value for at least one item, subtracting a property value for at least one item, and/or adding the property value (maximum value or minimum value) for at least one item.

In step 203, the recipe prediction unit 113 may predict a recipe including two or more materials containing at least one polymer for synthesizing the target polymer composite material and a mixing ratio for each of the two or more materials based on the at least one property and the recipe prediction model.

Here, the mixing ratio is a relative weight (or mass) ratio for each ofthe materials (e.g, a weight ratio sum (WRS)), and a content for each material may be represented as a percent (%).

To describe in more detail through FIG. 3, the recipe prediction unit 113 may input (301) at least one acquired property as an output variable of the recipe prediction model 121, calculate (303) two or more materials containing at least one polymer and a mixing ratio for each of the two or more materials as input variables of the recipe prediction model 121 so that the output variable is maximized and/or minimized as preset in at least one property, and determine (305) the calculated input variables as the recipe.

According to the description, the recipe prediction unit 113 may process the property values for each of two or more property items acquired based on at least one property as a maximum value and/or a minimum value as an output variable for the recipe prediction model 121, and acquire a recipe according to at least one property input from the recipe prediction model 121 as an input variable.

Here, the recipe output from the recipe prediction model 121 may include two or more materials containing at least one polymer and a mixing ratio for each of the two or more materials.

To describe in more detail, the recipe may include materials and a mixing ratio of the materials capable of producing a polymer composite material satisfying at least some properties acquired in step 201 as described above among various materials for producing a polymer composite material, such as at least one polymer, a reinforcing agent (e.g., talc), and an additive (e.g., polyolefin ether).

To this end, various materials that can be used for synthesizing the polymer composite material and information on the materials (e.g., property information) may be stored in the storage unit 120 as a database.

Here, the polymer may include polypropylene (PP), polyethylene (PE), or polyethylene terephthalate (PET).

In addition, at least some of the two or more materials included in the recipe output from the recipe prediction model 121 may include identification information (e.g., code, ID, etc.) of the corresponding materials and/or a property value for at least one property item.

Here, the recipe prediction model 121 may generate a recipe including identification information and/or property values for at least one property item for at least some of two or more materials included in the recipes, in order to specify any one material among various materials having identical or similar properties.

For example, the polymer may include at least one of a variety of recycled polymers, such as recycled polypropylene (rPP), recycled polyethylene (rPE), and recycled polyethylene terephthalate (rPET), as well as normal or virgin polymer.

At this time, even if it is the same polymer, especially in the case of the recycled polymers, the properties may not be uniform depending on the time of production, etc.

In order to distinguish between identical materials when there is a difference in the properties, the recipe may include identification information for materials including polymers and/or property values for at least one property item that can represent characteristics of the materials.

The recipe prediction model 121 for performing the procedure of step 203 may be generated based on at least one optimization algorithm and the property prediction model 123. To describe in more detail, the recipe model processing unit 117 may generate an objective function by applying at least one machine learning algorithm in the learning process of the property prediction model 123.

Here, the property prediction model 123 may be configured as a deep learning model configured to: input an input recipe including two or more materials containing at least one polymer and a mixing ratio for each of the two or more materials; and predict the output properties of the polymer composite material accordingto the input of the recipe and output them.

Based on this, the recipe model processing unit 117 may perform modelling of an objective function configured to input at least one property of a polymer composite material to be maximized and/or minimized in the learning process of the property prediction model 123 as an output variable, and output a recipe for synthesizing a polymer composite material having at least one maximized and/or minimized property as an input variable.

The recipe model processingunit 117 may evaluate the objective function using at least a portion of the dataset 125 configured to train the property prediction model 123 when performing modeling ofthe objective function.

Based on this, the recipe model processing unit 117 may perform modelling of the objective function by initializing the objective function and applying a Gaussian process thereto.

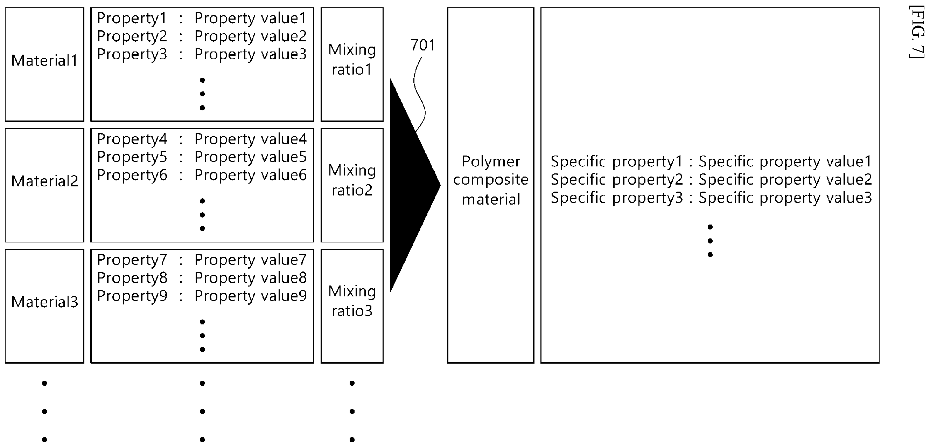

After describing the operations of FIGS. 2 to 6, the property prediction model 123, which is the basis of the recipe prediction model 121, and the training of the property prediction model 123 will be described in more detail through FIGS. 7 to 11.

In addition, the recipe model processing unit 117 may generate at least one relational expression for calculating a difference in properties between the pure polymer and the recycled polymer for at least one identical polymer in the learning process ofthe property prediction model 123.

To describe in more detail, the recipe model processing unit 117 may generate at least one relational expression configured to calculate a mixing ratio (or a change in the mixing ratio) of a recipe including a specific ratio of the recycled polymer compared to a recipe formed of the pure polymer, when the recipe is set to include a specific ratio of the recycled polymer.

In this regard, according to various embodiments, in step 201, the property information acquisition unit 111 acquires at least one property, as well as may acquire information on whether the polymer to be the material is formed of the pure polymer or formed by including the recycled polymer. At this time, the property information acquisition unit 111 may also acquire a ratio of the recycled polymer when the polymer to be the material is set to include the recycled polymer.

As described above, when the polymer forming the recipe is set to include a specific ratio of the recycled polymer, the recipe prediction unit 113 may generate a recipe by calculating (and/or reconstructing) the specific recycled polymer and the mixing ratio of the materials including the specific recycled polymer based on the relational expression.

To this end, the recipe prediction model 121 may be configured to predict two or more materials including polymers containing the pure polymer, and a mixing ratio of the two or more materials.

Returning to FIG. 2 again, as described above, the recipe prediction unit 113 may acquire a recipe including two or more materials containing at least one polymer and a mixing ratio for the materials by inputting at least one property acquired in step 201 into the recipe prediction model 121.

For example, the recipe prediction unit 113 may acquire a recipe including materials and a mixing ratio thereof, such as 30% of polyethylene, 20% of polypropylene, 15% of polyethylene terephthalate, 10% of polyurethane elastomer, and 5% of talc, etc.

In addition, for example when acquiring a recipe including the recycled polymer, the recipe prediction unit 113 may acquire a recipe including materials and a mixing ratio thereof such as 15% of polypropylene, 10% of polyethylene terephthalate, 5% of polyurethane elastomer, 10% of recycled polyethylene, 5% of recycled polypropylene, and 5% of talc, etc.

According to various embodiments, before performing the procedure of step 205, a verification operation of the recipe predicted through step 203 may be executed. For example, as shown in FIG. 4, the recipe prediction unit 113 may verify (401) the recipe based on the property prediction model.

To describe in more detail, when performing the verification step (401), the recipe prediction unit 113 may process the recipe as an input recipe to the property prediction model 123, then compare (501) an output property acquired from the property prediction model 123 with the at least one property, and determine (503) to output the recipe for the at least one property according to the comparison result.

At this time, the operation of comparing (501) the output property acquired from the property prediction model 123 with at least one property acquired in step 201 may include, as shown in FIG. 5, determining a difference value of at least some property values of one or more properties corresponding to at least some output property values, and comparing the difference value for each of the properties with a preset error.

The recipe prediction unit 113 may determine (601) whether the calculated difference value for at least some property items is within a preset error range for the corresponding property items, and if it is determined to be within the error range, determine (603) the corresponding recipe as a recipe for at least one property acquired in step 201 and output the results.

However, if it is determined that the calculated difference value for at least some property items deviates from the preset error range for the property items, the recipe prediction unit 113 may perform the step (203) of predicting the recipe again.

Here, when performing the step (203) of predicting the recipe again, the recipe prediction unit 113 may predict materials and a mixing ratio for the materials by changing at least some parameters of the recipe prediction model 121, for example, at least some parameters among the input variables.

Again, in step 205 of FIG. 2, the result output unit 115 may output the acquired recipe. To describe in more detail, the result output unit 115 may output result information including the recipe calculated as an input variable of the recipe prediction model 121.

The result output unit 115 may output result information through at least one display included in the device 100 and/or at least one display connected with the device 100.

At this time, if it is set to generate a recipe including the recycled polymer, the result output unit 115 may output the calculated recipe including the recycled polymer by including it in the result information.

According to various embodiments, the result output unit 115 may include verification results for the acquired recipe, for example, an error rate (%) for at least some property items in the result information.

In addition, the result output unit 115 may include at least some chemical formulas and/or at least some chemical structures of the polymer composite material acquired by inputting the predicted recipe into the property prediction model 123 in the result information.

According to various embodiments, the result output unit 115 may display an error rate (%) based on the difference value between the properties of the polymer composite material acquired as a result of inputting the predicted recipe into the property prediction model 123 and at least some properties acquired in step 201, through at least some chemical formulas and/or at least some chemical structures forming the polymer composite material. In this case, the result output unit 115 may display a portion corresponding to a specific property of the polymer composite material through highlighting or color change.

According to various embodiments, the result output unit 115 may transmit the result information to other preset devices through the communication unit.

When the operation of step 205 is completed, the result output unit 115 may terminate the procedures of embodiment in FIG. 2.

Hereinafter, the property prediction model 123 and the training of the property prediction model 123 will be described in detail through FIGS. 7 to 11.

According to the description, it is described that the recipe prediction model 121 may be generated by applying the machine learning algorithm in the learning process of the property prediction model 123.

At this time, the property prediction model 123 may be configured to input a recipe and predict properties of the polymer composite material according to the recipe, and/or may be configured to input a recipe and predict attributes of the materials included in the recipe, and then predict the properties of the polymer composite material according to the recipe based on the attributes of the materials.

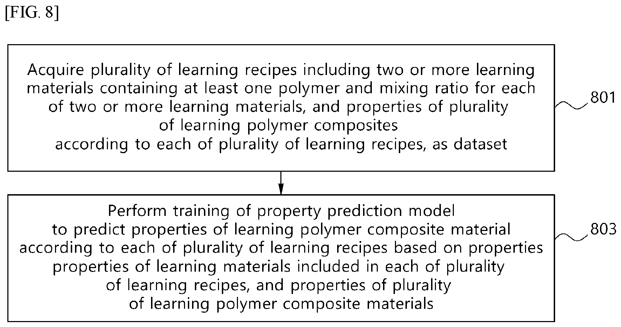

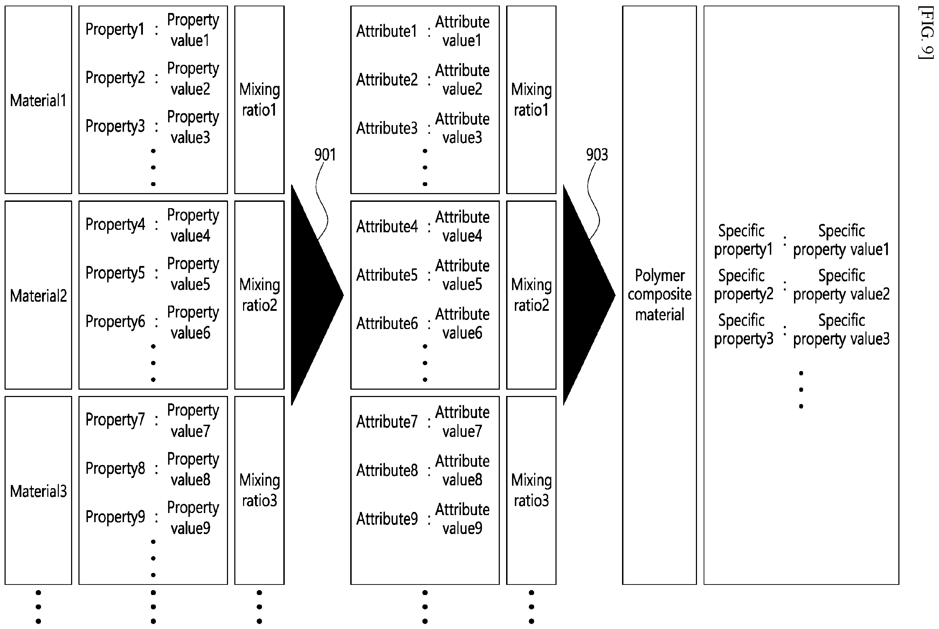

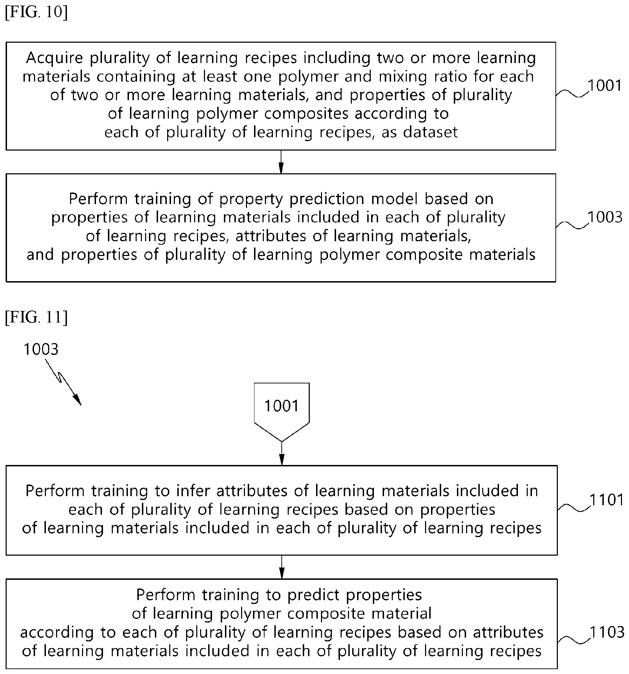

Hereinafter, the property prediction model configured to input a recipe and predict the properties of the polymer composite material according to the recipe, and the training of the property prediction model will be described in more detail through FIGS. 7 and 8. In addition, the property prediction model configured to input a recipe and predict the attributes of the materials included in the recipe, and then predict the properties of the polymer composite material according to the recipe based on the attributes of the materials, and the training of the property prediction model will be described in more detail through FIGS. 9 and 11.

First, the property prediction model and the training of the property prediction model will be described based on FIGS. 7 and 8.

FIG. 7 is a view schematically illustrating an operation of a property prediction model to output properties of the polymer composite material using the properties of the materials included in the recipe as an input in the device according to an embodiment. In addition, FIG. 8 is a flowchart illustrating the flow of an operation of training the property prediction model based on a deep learning algorithm in the device according to an embodiment.

F or the operation of the property prediction model, the recipe acquisition unit may input (or acquire) a recipe including two or more materials containing at least one polymer and a mixing ratio for each of the two or more materials.

According to an embodiment, the recipe acquisition unit may acquire a specific recipe through at least one input unit (not shown) and/or communication unit (not shown) included in the device 100.

According to an embodiment, the properties for each of the materials stored in the recipe may include at least one property of tensile strength, tensile failure, tearing strength, yield strength, flexural modulus, impact strength, elongation at break, heat distortion temperature, air permeability and shrinkage, and property values corresponding thereto.

In addition, the recipe may include information on properties for each of the materials included therein. However, the properties for each of the materials may be stored in the storage unit 120, and in this state, the recipe acquisition unit may acquire the properties for each of the materials from the storage unit 120.

To describe the materials acquired from a specific recipe, properties of the materials, and a mixing ratio thereof in more detail with reference to FIG. 7, the recipe acquisition unit may acquire various materials for forming a polymer composite material, including material 1, material 2, and material 3, from the acquired specific recipe.

At this time, the recipe acquisition unit may acquire identification codes corresponding to materials from the recipe, and also acquire information on materials corresponding to each identification code from the storage unit 120.

Referring to FIG. 7, it illustrates that the materials included in the recipe are three materials, such as material 1, material 2, and material 3, but it is not limited thereto, and the recipe may also include at least one more or less material than three.

In addition, the recipe acquisition unit may acquire the properties for each of the materials included in the recipe.

For example, the recipe acquisition unit may acquire properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3.

Here, referring to FIG. 7, it illustrates that, for the properties included in each of the materials included in the recipe, three properties are included therein, but it is not limited thereto, and the properties for each of the materials may also include at least one more or less property than three.

Here, the expressions of properties 1 to 9 are expressions for describing the properties possessed by the materials, and two or more properties among properties 1 to 9 may represent the same property. Similarly, two or more among mixing ratios 1 to 3 may represent the same mixing ratio.

In addition, the recipe acquisition unit may acquire the mixing ratio for each of the materials included in the recipe.

For example, the recipe acquisition unit may acquire a mixing ratio 1 for material 1, a mixing ratio 2 for material 2, and a mixing ratio 3 for material 3. To describe in more detail, the mixing ratios of the materials included in the recipe may be included as a ratio possessed by each of the materials based on 100% oftotal materials, such as a mixing ratio of 20% for material 1, a mixing ratio of 30% for material 2, and a mixing ratio of 50% for material 3.

Thereafter, the property prediction unit may predict the properties of the polymer composite material according to the recipe based on the recipe and the property prediction models.

According to an embodiment, the property prediction unit may process a plurality of properties for each of two or more materials acquired based on the recipe as an input to the property prediction model, and acquire properties of the polymer composite material according to the recipe as an output from the property prediction model.

To describe in more detail with reference to FIG. 7, the property prediction model may include a property-property prediction model (hereinafter, a property prediction model 701) trained to predict the properties of the polymer composite material according to the recipe based on the properties of the materials included in the recipe.

The property prediction unit may process the materials included in the recipe (e.g, material 1, material 2, and material 3), the properties of the materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and the mixing ratios for the materials (e.g., mixing ratio 1 for material 1, mixing ratio 2 for material 2, and mixing ratio 3 for material 3) as inputs to the property prediction model 701, and acquire specific properties of the polymer composite material according to the recipe (e.g, specific property value 1 for specific property 1, specific property value 2 for specific property 2, and specific property value 3 for a specific property 3).

Here, the specific property may include at least one property of melt index, tensile strength, tensile failure, tearing strength, yield strength, flexural modulus, impact strength, elongation at break, heat distortion temperature, air permeability and shrinkage.

Here, referring to FIG. 7, it is described that, for the properties ofthe polymer composite material output by the property prediction model 701, three specific properties are output, but it is not limited thereto, and the properties ofthe polymer composite material output by the property prediction model 701 may further include at least one more or less specific property than three.

The pre-trained property prediction model 701 may be a deep learning model trained to output the specific properties of the polymer composite material according to the recipe based on the properties ofthe materials included in the recipe.

In this regard, the training of the property prediction model 701 will be described in detail through FIG. 8.

First, the property prediction model 701 may be configured to perform training based on at least some of various deep learning algorithms which process structured data, such as TabNet, XGBoost, LightGBM, CatBoost, and deep neural network (DNN).

The property prediction model 701 may include a deep learning model configured to input recipes including a plurality of materials, properties for each ofthe materials, and a mixing ratio for the plurality of materials, and output properties of the polymer composite material corresponding to the recipes.

In step 801, the model learning unit may acquire a plurality of learning recipes including two or more learning materials containing at least one polymer and a mixing ratio for each of the two or more learning materials, and properties of a plurality of learning polymer composite materials according to each of the plurality of learning recipes, as a dataset.

To describe in more detail, the dataset may include the plurality of learning recipes and the properties of the learning polymer composite materials according to each of the plurality of learning recipes. Here, each ofthe plurality of recipes may include information on two or more materials, properties of each of the two or more materials, and a mixing ratio of each of the two or more materials.

For example, to describe the dataset including information on a specific learning recipe and properties of the specific learning polymer composite material according to the specific learning recipe, it may include learning materials included in the specific learning recipe (e.g, material 4, material 5, and material 6), properties for the learning materials (e.g., properties including property value 10 for property 10, property value 11 for property 11, and property value 12 for property 12 in material 4, properties including property value 13 for property 13, property value 14 for property 14, and property value 15 for property 15 in material 5, and properties including property value 16 for property 16, property value 17 for property 17, and property value 18 for property 18 in material 6), and mixing ratios for the learning materials (e.g., mixing ratio 4 for material 4, mixing ratio 5 for material 5, and mixing ratio 6 for material 6).

The specific learning polymer composite material according to the specific learning recipe is a polymer composite material which is confirmed to be capable of being synthesized using the specific learning recipe, and may include properties possessed by the specific learning polymer composite material (e.g., properties including property value 19 for property 19, property value 20 for property 20, and property value 21 for property 21).

Here, it is described that the number of materials included in the learning recipe, the number of properties included in each of the materials, and the number of properties of the learning polymer composite material are three, respectively, but they are not limited thereto, and may be changed depending on the setting.

In addition, the expressions of properties 10 to 18 are expressions for describing the properties possessed by the materials, and two or more properties among properties 10 to 18 may represent the same property, and at least one property among properties 10 to 18 may represent the same property as at least one property among properties 1 to 9 and property 19 to property 21.

Similarly, two or more of the mixing ratios 4 to 6 may have the same mixing ratio, and at least one of the mixing ratios 4 to 6 may have the same value as at least one of the mixing ratios 1 to 3.

Here, a dataset 125 and/or data included in the dataset (e.g., recipes and properties of the polymer composite material corresponding to the recipes) may be received by the recipe acquisition unit through an input unit or the communication unit.

In step 803, the model learning unit may perform training of the property prediction model to predict the properties of the learning polymer composite material according to each of the plurality of learning recipes based on the properties of the learning materials included in each of the plurality of learning recipes, and the properties of the plurality of learning polymer composite materials.

To describe in more detail, the model learning unit may perform training of the property prediction model 701 so as to, when inputting any one recipe, output the properties ofthe polymer composite material according to the corresponding recipe, based on the learning materials included in each of the plurality of learning recipes, properties of the learning materials, and properties of the plurality of learning polymer composite materials.

When performing training of the property prediction model 701, the model learning unit may train the property prediction model 701 based on the specific properties of at least some specific materials included in the learning recipe, and specific properties of the learning polymer composite material.

To describe in more detail, the model learning unit may extract, from each of the plurality of learning recipes, a first preset learning specific property among properties possessed by each of at least one learning specific material, and a second preset learning specific property among the properties ofthe learning polymer composite material.

To this end, the device 100 may further include a property extraction unit for extracting the properties of the materials included in the recipes and/or specific properties from the properties of the polymer composite material.

Here, the specific material may include a polymer, talc, or a polyolefin elastomer.

Here, the model learning unit and/or the property extraction unit may extract a first learning specific property or a second learning specific property including at least some of melt index, tensile strength, tensile failure, tearing strength, yield strength, flexural modulus, impact strength, elongation at break, heat distortion temperature, air permeability and shrinkage.

Here, the second learning specific property may include at least one property among the specific properties of the polymer composite material to be predicted through the pre-trained property prediction model 123.

In addition, the model learning unit and/or the property extraction unit may extract the first learning specific property in different manners based on types of the learning specific material.

For example, when the learning specific material is polypropylene (PP), the model learning unit and/or the property extraction unit may extract at least one property among the melt index, flexural modulus, tensile strength, impact strength, shrinkage, and heat distortion temperature as a first feature.

When the learning specific material is polyethylene (PE), the model learning unit and/or the property extraction unit may extract at least one property among the impact strength, tearing strength, yield strength, tensile failure, and elongation at break as a second feature.

In addition, when the learning specific material is polyethylene terephthalate (PET), the model learning unit and/or the property extraction unit may extract at least one property among the air permeability, tensile strength, tearing strength, and elongation at break as a third feature.

Thereafter, the model learning unit may perform training of the property prediction model for each of the plurality of learning recipes based on the plurality of learning recipes including the plurality of first learning specific properties and the second learning specific properties.

To describe in more detail, the model learning unit may perform training of the property prediction model 701 to output properties including the second learning specific properties ofthe specific polymer composite material corresponding to the specific learning recipe by setting the first learning specific properties and/or the second learning specific properties as a region of interest, and using the specific learning recipe and the first learning specific properties of the specific learning recipe as inputs.

The property prediction unit may perform an operation of predicting the properties of the polymer composite material for the recipe using the property prediction model 701 trained as described above.

The property prediction unit may process two or more materials included in the recipe, properties for each of the materials, and a mixing ratio of each of the materials as inputs to the property prediction model 701, and acquire properties of the polymer composite material from the property prediction model 701.

According to various embodiments, the property prediction unit may extract at least one specific property among the plurality of properties possessed by each of at least one specific material among two or more materials included in the input recipe, and process it as an input to the property prediction model 701.

At this time, the property prediction unit may predict the properties of the polymer composite material using the two or more materials and the at least one specific property of the at least one specific material as inputs to the property prediction model 701.

The result output unit may output the properties of the polymer composite material. To describe in more detail, the result output unit may generate result information by including the acquired recipe and the specific properties of the polymer composite material acquired from the property prediction model 701 corresponding to the acquired recipe, and output the result information.

According to various embodiments, the result output unit may include at least some chemical formulas and/or at least some chemical structures of the polymer composite material predicted by the pre-trained property prediction model 123 corresponding to the recipe in the result information.

In addition, the result output unit may display a portion corresponding to the specific properties of the predicted polymer composite material through at least some chemical formulas and/or at least some chemical structures. In this case, the result output unit may display the portion corresponding to the specific property of the polymer composite material through highlighting or color change.

According to various embodiments, the result output unit may transmit the result information to other preset devices through the communication unit.

Thereafter, the property prediction model and the training of the property prediction model will be described based on FIGS. 9 and 11.

FIG. 9 is a view schematically illustrating an operation of the property prediction model to predict attributes of the materials using the properties of the materials included in the recipe as an input, and output properties of the polymer composite material using the attributes of the materials as an input in the device according to an embodiment. FIG. 10 is a flowchart illustrating the flow of an operation of training the property prediction model based on the deep learning algorithm in the device according to an embodiment of the present disclosure. In addition, FIG. 11 is a flowchart illustrating the flow of the detailed operation of training the property prediction model based on the deep learning algorithm in the device according to an embodiment of the present disclosure.

For the operation of the property prediction model, the recipe acquisition unit may input (or acquire) a recipe including two or more materials containing at least one polymer and a mixing ratio for each of the two or more materials.

The materials acquired from the specific recipe, the properties of the materials, and the mixing ratio thereof have been described in detail through FIGS. 7 and 8, and therefore the operation of acquiring various materials, the properties of the materials, and the mixing ratio of the materials for forming the polymer composite material including material 1, material 2, and material 3 from the specific recipe by the recipe acquisition unit will not be described.

Thereafter, the property prediction unit may predict the attributes of the materials based on the properties of the materials acquired from the recipe, and may predict the properties ofthe polymer composite material based on the attributes of the materials.

For this purpose, to describe in more detail with reference to FIG. 9, the property prediction model may include a property-attribute prediction model (hereinafter, a first prediction model 901) configured to predict attributes of the materials using the properties of materials as an input, and an attribute- property prediction model (hereinafter, a second prediction model 903) configured to predict specific properties of the polymer composite material using the attributes of the materials.

The property prediction unit may predict at least one attribute for each of the two or more materials based on the two or more materials and the first prediction model 901.

According to an embodiment, the property prediction unit may process a plurality of properties for each of two or more materials acquired based on the recipe as an input to the first prediction model 901, and acquire a plurality of attributes for each of the two or more materials from the first prediction model 901 as an output.

The property prediction unit may process materials included in the recipe (e.g., material 1, material 2, and material 3), properties ofthe materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and mixing ratios for the materials (e.g., mixing ratio 1 for material 1, mixing ratio 2 for material 2, and mixing ratio 3 for material 3) as inputs to the first prediction model 901, and acquire attributes of the materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3).

Here, the attributes of the materials may include at least one attribute of syndiotactic PP content or molecular weight, atacticPP content or molecular weight, rubber content or molecular weight, type or content of reinforcing agent, PE content or molecular weight, type or content of copolymer, long-chain branch content, acid content, density, and degree of crystallization.

Here, referring to FIG. 9, it is described that, for each of the attributes of the materials output by the first prediction model 901, three attributes are output, but it is not limited thereto, and each of the attributes of the materials output by the first prediction model 901 may include at least one more or less attribute than three.

The property prediction unit may predict the properties of the polymer composite material according to the recipe based on the at least one attribute for each of the two or more materials and the property prediction model.

According to an embodiment, the property prediction unit may process the attributes of the materials and the mixing ratio of the materials acquired from the first prediction model 901 as inputs to the second prediction model 903, and acquire the properties ofthe polymer composite material according to the recipe from the second prediction model 903 as an output.

The property prediction unit may process the attributes for materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3) as an input to the second prediction model 903, and acquire specific properties ofthe polymer composite material according to the recipe (e.g., properties including specific property value 1 for specific property 1, specific property value 2 for specific property 2, and specific property value 3 for specific property 3).

Here, the specific property may include at least one property of melt index, tensile strength, tensile failure, tearing strength, yield strength, flexural modulus, impact strength, elongation at break, heat distortion temperature, air permeability and shrinkage.

Here, referring to FIG. 9, it is described that, for the properties ofthe polymer composite material output by the second prediction model 903, three specific properties are output, but it is not limited thereto, and the properties of the polymer composite material output by the second prediction model 903 may further include at least one more or less specific property than three.

The pre-trained first prediction model 901 may be a deep learning model trained to output attributes of the materials based on the properties of the materials included in the recipe, and the pre-trained second prediction model 903 may be a deep learning model trained to output specific properties of the polymer composite material according to the recipe based on the attributes of the materials.

In this regard, the training of the first prediction model 901 and the second prediction model 903 will be described in detail through FIGS. 10 and 11.

First, the first prediction model 901 and the second prediction model 903 may be configured to perform training based on at least some of various deep learning algorithms which process structured data, such as TabNet, XGBoost, LightGBM, CatBoost, and deep neural network (DNN).

In step 1001, the model learning unit may acquire a plurality of learning recipes including two or more learning materials containing at least one polymer and a mixing ratio for each of the two or more learning materials, and properties of a plurality of learning polymer composite materials according to each of the plurality of learning recipes, as a dataset.

To describe in more detail, the dataset may include the plurality of learning recipes and the properties of the learning polymer composite materials according to each of the plurality of learning recipes. Here, each of the plurality of recipes may include information on two or more materials, properties of each of the two or more materials, attributes of each of the two or more materials, and a mixing ratio of each of the two or more materials.

For example, to describe the dataset including information on a specific learning recipe and properties of the specific learning polymer composite material according to the specific learning recipe, it may include learning materials included in the specific learning recipe (e.g, material 4, material 5, and material 6), properties for the learning materials (e.g., properties including property value 10 for property 10, property value 11 for property 11, and property value 12 for property 12 in material 4, properties including property value 13 for property 13, property value 14 for property 14, and property value 15 for property 15 in material 5, and properties including property value 16 for property 16, property value 17 for property 17, and property value 18 for property 18 in material 6), attributes for the learning materials (e.g., attributes including attribute value 10 for attribute 10, attribute value 11 for attribute 11, and attribute value 12 for attribute 12 in material 4, attributes including attribute value 13 for attribute 13, attribute value 14 for attribute 14, and attribute value 15 for attribute 15 in material 5, and attributes including attribute value 16 for attribute 16, attribute value 17 for attribute 17, and attribute value 18 for attribute 18 in material 6), and mixing ratios for the learning materials (e.g., mixing ratio 4 for material 4, mixing ratio 5 for material 5, and mixing ratio 6 for material 6).

The specific learning polymer composite material according to the specific learning recipe is a polymer composite material which is confirmed to be capable of being synthesized using the specific learning recipe, and may include properties possessed by the specific learning polymer composite material (e.g., properties including property value 19 for property 19, property value 20 for property 20, and property value 21 for property 21).

Here, it is described that the number of materials included in the learning recipe, the number of properties included in each of the materials, and the number of properties of the learning polymer composite material are three, respectively, but they are not limited thereto, and may be changed depending on the setting.

In addition, the expressions of properties 10 to 18 are expressions for describing the properties possessed by the materials, and two or more properties among properties 10 to 18 may represent the same property, and at least one property among properties 10 to 18 may represent the same property as at least one property among properties 1 to 9 and property 19 to property 21.

Similarly, two or more of the mixing ratios 4 to 6 may have the same mixing ratio, and at least one of the mixing ratios 4 to 6 may have the same value as at least one of the mixing ratios 1 to 3.

Here, the dataset 125 and/or data included in the dataset (e.g., recipes and properties of the polymer composite material corresponding to the recipes) may be received by the recipe acquisition unit through an input unit or the communication unit.

In step 1003, the model learning unit may perform training of the property prediction model based on properties of the learning materials included in each of the plurality of learning recipes, attributes of the learning materials, and the properties ofthe plurality of learning polymer composite materials.

In this regard, the operation of training the first prediction model 901 and the second prediction model 903 will be described in more detail with reference to FIG. 11. At least some of the procedures in steps 1101 and/or 1103 of FIG. 11 may be performed in step 1003 of FIG. 10.

In step 1101, the model learning unit may perform training of the first prediction model 901 to infer attributes ofthe learning materials included in each of the plurality of learning recipes based on the properties of the learning materials included in each of the plurality of learning recipes.

To describe in more detail, when inputting any one recipe based on the learning materials included in each of the plurality of learning recipes and the properties of the learning materials, the model learning unit may perform training of the first prediction model 901 to output the attributes of the materials included in the recipe.

When performing training of the first prediction model 901, the model learning unit may train the first prediction model 901 based on the specific properties of at least some specific materials included in the learning recipes.

To describe in more detail, the model learning unit may extract, from each of the plurality of learning recipes, a first preset learning specific property among properties possessed by each of at least one learning specific material, and a second preset learning specific property among the properties ofthe learning polymer composite material.

To this end, the device 100 may further include a property extraction unit for extracting the properties of the materials included in the recipes and/or specific properties from the properties of the polymer composite material.

Here, the specific material may include a polymer, talc, or a polyolefin elastomer.

Here, the model learning unit and/or the property extraction unit may extract a first learning specific property or a second learning specific property including at least some of melt index, tensile strength, tensile failure, tearing strength, yield strength, flexural modulus, impact strength, elongation at break, heat distortion temperature, air permeability and shrinkage.

Here, the second learning specific property may include at least one property among the specific properties of the polymer composite material to be predicted through the pre-trained property prediction model 123.

In addition, the model learning unit and/or the property extraction unit may extract the first learning specific property in different manners based on types of the learning specific material.

For example, when the learning specific material is polypropylene (PP), the model learning unit and/or the property extraction unit may extract at least one property among the melt index, flexural modulus, tensile strength, impact strength, shrinkage, and heat distortion temperature as a first feature.

When the learning specific material is polyethylene (PE), the model learning unit and/or the property extraction unit may extract at least one property among the impact strength, tearing strength, yield strength, tensile failure, and elongation at break as a second feature.

In addition, when the learning specific material is polyethylene terephthalate (PET), the model learning unit and/or the property extraction unit may extract at least one property among the air permeability, tensile strength, tearing strength, and elongation at break as a third feature.

Thereafter, the model learning unit may perform training of the first prediction model 901 to infer attributes of the learning materials included in each of the plurality of learning recipes based on the first learning specific properties included in each of the plurality of learning recipes.

To describe in more detail, the model learning unit may perform training of the first prediction model 901 to output attributes for at least some materials including specific materials among the materials included in the specific learning recipe by setting the first learning specific property as a region of interest, and using the specific learning recipe and the first learning specific property of the specific learning recipe as inputs.

In step 1103, the model learning unit may perform training of the second prediction model 903 to predict properties of the learning polymer composite material according to each of the plurality of learning recipes based on the attributes of the learning materials included in each of the plurality of learning recipes.

To describe in more detail, when inputting properties of at least some materials included in any one recipe based on the learning materials included in each of a plurality of learning recipes and attributes of the learning materials, the model learning unit may perform training of the second prediction model 903 to output properties of the polymer composite material according to the recipes.

When performing training of the second prediction model 903, the model learning unit may train the second prediction model 903 based on the plurality of materials included in the learning recipe and attributes of at least some specific materials.

Thereafter, the model learning unit may perform training of the second prediction model 903 to predict the second specific property of the learning polymer composite material according to each of the plurality of learning recipes based on the attributes of the learning materials included in each of the plurality of learning recipes, and the second preset learning specific property among the properties of the learning polymer composite material.

To describe in more detail, the model learning unit may perform training of the second prediction model 903 to output properties including the second learning specific property of the specific polymer composite material corresponding to the specific learning recipe by setting the second learning specific property as a region of interest, and using the specific learning recipe, the attributes for at least some including specific materials among the materials included in the specific learning recipe, and the second learning specific property of the specific learning recipe as inputs.

The property prediction unit may perform an operation of predicting the properties of the polymer composite material for the recipe using the property prediction model 123 including the first prediction model 901 and the second prediction model 903, which are trained as described above.

An operation of predicting the properties of the polymer composite material according to the input recipe may be performed using the trained first prediction model 901 and/or second prediction model 903.

The property prediction unit may process two or more materials included in the recipe, properties for each of the materials, and a mixing ratio of each of the materials as inputs to the first prediction model 901, and acquire attributes for at least some of the materials included in the recipe from the first prediction model 901.

Here, the property prediction unit may extract at least one specific property among the plurality of properties possessed by each of at least one specific material among two or more materials included in the input recipe, and process it as an input to the first prediction model 901.

At this time, the property prediction unit may predict attributes for at least some of the materials included in the recipe using the two or more materials and the at least one specific property for the at least one specific material as inputs to the first prediction model 901.

Thereafter, the property prediction unit may predict the properties of the polymer composite material according to the recipe as described above based on the attributes of the materials acquired from the first prediction model 901 and the input recipe.

The result output unit may output the properties of the polymer composite material. To describe in more detail, the result output unit may generate result information by including the recipe acquired in step 701 and the specific properties of the polymer composite material acquired from the property prediction model 701 corresponding to the acquired recipe, and output the result information. Here, the result output unit may further include the predicted attributes of the materials included in the recipe in the result information.

According to various embodiments, the result output unit may include at least some chemical formulas and/or at least some chemical structures of the polymer composite material predicted by the pre-trained property prediction model 123 corresponding to the recipe in the result information.

In addition, the result output unit may display the portion corresponding to the specific property of the predicted polymer composite material through at least some chemical formulas and/or at least some chemical structures. In this case, the result output unit may display the portion corresponding to the specific property of the polymer composite material through highlighting or color change.

According to various embodiments, the result output unit may transmit the result information to other preset devices through the communication unit.

The recipe prediction model 121 may be generated by applying a machine learning algorithm in the above-described learning process of the property prediction model 701 and/or in the learning process of the first prediction model 901 and the second prediction model 903.

As described above, the method for generating recipes of a polymer composite material and the device thereof may provide an environment capable of quickly checking materials and a mixing ratio thereof for a polymer composite material only by inputting at least some properties of the desired polymer composite material.

According to various embodiments, the method for generating recipes of a polymer composite material and the device thereof allow users to easily convert and check a material mixing ratio when using a pure polymer or a material mixing ratio when including a recycled polymer by inputting the properties of the target polymer composite material, thereby significantly reducing the time and costs required for development of materials.

According to various embodiments, two or more of the property information acquisition unit 111, the recipe prediction unit 113, the result output unit 115, and the recipe model processing unit 117 may be formed as one module, or each unit may be formed as a separate module. In this case, each module may include at least one processor.

According to various embodiments, the functions of various embodiments described as being performed by the device 100 are operations processed through the processing unit 110 of the device 100, and may be performed by organically being connected to the device 100 and/or components of the device connected to the device 100.

To this end, at least some of the property information acquisition unit 111, the recipe prediction unit 113, the result output unit 115, and the recipe model processing unit 117 may be formed to be included in the processing unit 110 as shown in FIG. 1.

The processing unit 110 includes at least one processor, and may process data through at least one program (application, tool, plug-in, software, etc.).

The storage unit 120 may store various data processed by at least one component ofthe device 100 (e.g., the processing unit 110, the communication unit or the like). The data may include, for example, a program for processing control commands, data processed through the program, or input data and output data related thereto.

The communication unit (not shown) may support establishment of a wired communication channel or establishment of a wireless communication channel inside the device 100 and/or between the device 100 and at least one other device (e.g., the user device or a server), and performing communication through the established communication channel.

The input/output unit may include or be connected to at least some of an input unit (not shown) for inputting data, such as a keyboard, mouse, or touch pad, and an output unit (not shown) for outputting data, such as a display unit (e.g., display), speaker, or driving unit.

According to various embodiments of the present disclosure, the device 100 or user device may include at least some of the functions in the range of all information and communication devices, including a mobile terminal, a multimedia terminal, a wired terminal, a fixed terminal, and an internet protocol (IP) terminal.

The device 100 is a device for processing the control commands, and may include at least some of the functions of a workstation or a large-scale database, or may be connected therewith through communication.

As described above, although the embodiments have been described with reference to the limited drawings, it will be apparent to those skilled in the art that various modifications and alternations may be applied thereto based on the various embodiments.

For example, adequate effects or results may be achieved even if the foregoing processes and methods are carried out in different order than those described above, and/or the above-described elements, such as systems, structures, devices, or circuits, are combined or coupled in different forms and modes than those described above, or substituted or switched with other components or equivalents.

In particular, when describing with reference to the flowchart, it is described that a plurality of steps are configured and the steps are sequentially executed in a designated order, but it is not necessarily limited to the designated order.

In other words, executing by changing or deleting at least some of the steps described in the flowchart or adding at least one step is applicable as an embodiment, and executing one or more steps in parallel may also be applicable as an embodiment. That is, it is not limited to that the steps are necessarily operated in a time-series order, and should be included in various embodiments of the present disclosure.

Therefore, other implements, other embodiments, and equivalents to claims are within the scope of claims to be described below.

## Claims

1. A method for predicting recipes of a polymer composite material, the method comprising:
obtaining at least one property for a target polymer composite material;
predicting a recipe including at least two materials containing at least one polymer for synthesizing the target polymer composite material and a mixing ratio for each of the two or more materials based on at least one property and recipe prediction model (123; 121); and
outputting the recipe.

2. The method according to claim 1, wherein the recipe prediction model (121) includes a function configured to set properties of the polymer composite material, which is an output of the property prediction model (123), as an output variable to be maximized or minimized based on a machine learning algorithm, and predict a recipe, which is an input of the property prediction model (123), as an input variable, and
the property prediction model (123) includes an artificial intelligence model trained to input an input recipe including two or more materials containing at least one polymer and a mixing ratio for each of the two or more materials, and predict output properties of the polymer composite material according to an input of the input recipe and output them.

3. The method according to claim 1 or 2, wherein the step of predicting a recipe comprises:
inputting the at least one property into an output variable of the recipe prediction model (121);
calculating the two or more materials containing at least one polymer and a mixing ratio for each of the two or more materials as input variables of the recipe prediction model (121) so that the output variable is maximized or minimized; and
determining the calculated input variables as the recipe.

4. The method according to anyone of the preceding claims, wherein the step of predicting generates the recipe by including at least a portion of a first recipe including a pure polymer and a second recipe including at least one recycled polymer.

5. The method according to claim 4, wherein, when generating the second recipe, the step of predicting a recipe reconstructs a mixing ratio for each of two or more materials including the recycled polymer based on a relational expression between a difference in properties of the pure polymer and the recycled polymer.

6. The method according to claim 5, wherein the relational expression for a difference in properties is generated in a learning process of the property prediction model (123) on which the recipe prediction model (121) is based, and
the property prediction model (123) includes an artificial intelligence model trained to input an input recipe including two or more materials containing at least one polymer and a mixing ratio for each of the two or more materials, and predict output properties of the polymer composite material according to an input of the input recipe and output them.

7. The method according to claim 2 or any claim depending thereon, further comprising verifying the recipe predicted from the recipe prediction model (121) based on the property prediction model (123), which is performed between the predicting step and the outputting step.

8. The method according to claim 7, wherein the step of verifying the recipe comprises:
processing the recipe as an input recipe to the property prediction model (123), and then comparing the output properties acquired from the property prediction model (123) with at least one property; and
determining to output the recipe for the at least one property according to the comparison result.

9. The method according to claim 8, wherein the step of determining to output the recipe comprises:
determining the recipe as a recipe for the at least one property if the comparison result is within a preset error range; and performing the prediction step again if the comparison result deviates from the preset error range.

10. The method according to anyone of the preceding claims, wherein the at least one property includes a value for at least one item of melt index, tensile strength, tensile failure, tearing strength, yield strength, flexural modulus, impact strength, elongation at break, heat distortion temperature, air permeability and shrinkage, and
the at least one polymer includes polypropylene (PP), polyethylene (PE), or polyethylene terephthalate (PET).

11. A device for predicting recipes of a polymer composite material, the device comprising:
a property information acquisition unit (111) configured to acquire at least one property of a target polymer composite material;
a recipe prediction unit (113) configured to predict a recipe including two or more materials containing at least one polymer for synthesizing the target polymer composite material and a mixing ratio for each of the two or more materials based on at least one property and recipe prediction model (123; 121); and
a result output unit (115) configured to output the recipe.

12. The device according to claim 11, wherein the recipe prediction model (121) is a function configured to set properties of the polymer composite material, which is an output of the property prediction model (123), as an output variable to be maximized or minimized based on a machine learning algorithm, and predict a recipe, which is an input of the property prediction model (123), as an input variable, and
the property prediction model (123) includes an artificial intelligence model trained to input an input recipe including two or more materials containing at least one polymer and a mixing ratio for each of the two or more materials, and predict output properties of the polymer composite material according to an input of the input recipe and output them.

13. The device according to claim 11 or 12, wherein the recipe prediction unit (113) is configured to input the at least one property into an output variable of the recipe prediction model (121), to calculate two or more materials containing at least one polymer and a mixing ratio for each of the two or more materials as input variables of the recipe prediction model (121) so that the output variable is maximized or minimized, and to determine the calculated input variable as the recipe.

14. The device according to anyone of claims 11-13, wherein the recipe prediction unit (113) is configured to generate the recipe by including at least a portion of a first recipe including a pure polymer and a second recipe including at least one recycled polymer.

15. The device according to claim 14, wherein the recipe prediction unit (113) is configured, when generating the second recipe, to reconstruct the mixing ratio for each of two or more materials including the recycled polymer based on a relational expression between a difference in properties of the pure polymer and the recycled polymer.
